# EUROPEAN PATENT SPECIFICATION

(11) **EP 4 303 290 B1**
(45) Date of publication and mention of the grant of the patent: **12.03.2025**
(21) Application number: 22215517.8
(22) Date of filing: 21.12.2022
(51) Int. Cl.: C11D 17/04, C11D 3/00

(54) **READILY DISSOLVABLE CARTRIDGE COMPLEX AND INDUSTRIAL SYSTEMS AND PROCESSES OF FABRICATING THE SAME**
LEICHT LÖSLICHER KARTUSCHENKOMPLEX SOWIE INDUSTRIELLE SYSTEME UND VERFAHREN ZUR HERSTELLUNG DAVON
COMPLEXE DE CARTOUCHE FACILEMENT SOLUBLE ET SYSTÈMES INDUSTRIELS ET PROCÉDÉS DE FABRICATION ASSOCIÉS

(30) Priority: 05.07.2022 IL 29453922
(43) Date of publication of application: 10.01.2024
(73) Proprietor: Capsule Minimal Ltd., 1080500 Kibuts Ein Ha' Natziv (IL)
(72) Inventor: KRUPIK, Amichai, 4640525 Herzeliya (IL)
(74) Representative: Pearl Cohen Zedek Latzer Baratz UK LLP

(56) References cited:
- EP-A2- 0 903 405
- US-A- 4 099 912
- US-A1- 2005 075 259
- US-B2- 10 569 286
- US-B2- 8 814 456

## Description

### TECHNICAL FIELD

In general, the present invention pertains to the art of industrial chemistry. In particular, the invention relates to a readily dissolvable cartridge complex as well as processes of fabricating the same.

### BACKGROUND ART

It is believed that the current state of the art is represented by the following patent literature: US8119587, US8476211, US8697621, US8980816, US10016098, US10485739, US10526570, US10894005, US11236293, US2016101204, US2018216053, US2020102524, KR20130003570 and WO2021174183. Further relevant art includes US2005075259A1 and US 1056928682.

Hygiene products industry, producing liquid soaps, body wash, hair care products and many other cosmetics is one of the most polluting. One unit of a liquid soap or shampoo product typically contains: a highly pollutive disposable plastic bottle, about 20 percent of active ingredients, some of which are environmental and health-harming and about 80 percent water. Most of the common production processes are environmentally harming, employing pollutive ships, trucks and warehouses, environmentally harmful labels and packaging materials. That unproportionable investment in packaging materials and water transportation is environmentally unsustainable.

Millions of liquid soap, shampoo and other hair or body care products in bottles are being sold every day around the world. Those plastic bottles require high energy consumption and other polluting inputs to be produced. Further to that, plastic bottles are to be disposed of after a short utilization. 245 million people in the United States used body wash in disposable bottles in 2020. According to research published by Statista Research department in 2021, approximately 1.5 billion disposable bottles of body wash are disposed every year, not including other products such as shampoos, conditioners, mouth wash, etc.

Plastic bottles production is one of the most pollutive industries and major environmental problems as of today. An environmental harming component, as of its initial production from fossil fuels, through its logistics and transportation and up to its disposal. Further to the direct pollution involved with transportation, there are many indirect mass transportation pollution processes, such as ships, trucks and containers manufacturing, packaging production, tiers, cranes, forklifts, etc. A direct pollutive process is the inefficient transport and storage of the empty plastic bottles (from the plastic factory to the soap factory and when disposed of after use), as a full trailer loaded with bottles will carry more than 95 percent air due to the bottles high volume. Warehouses involve highly pollutive direct and indirect processes. From giant electric power consuming warehouses built from pollutive construction materials, through logistic equipment such as cranes and forklifts to disposable packaging materials (stretch plastic, cardboard boxes, etc.), as well as fixed packaging and containing facilities (shelves, containers, etc.).

Disposing of plastic bottles is also one of the most harmful activities to the environment, polluting air, water and soil. Plastic bottles remain through hundreds of years, going through microplastic particles which are air and water carriable and virtually impossible to detect and/or clean. Disposed bottles also typically include active ingredient residues that further pollute soil and water sources. Moreover, each unit of a liquid soap or shampoo product typically contains packaging and labels. Plastic stretch films, cardboard cases, plastic, and paper labels are harmful to the environment in three main ways: direct and indirect pollutive production process; direct and indirect transportation production to and from the soap plant as well as to and the consumer, when disposed of; highly pollutive disposal, contaminating soil, water and air. Moreover and perhaps more importantly, each unit of a liquid soap or shampoo product typically contains about 80 percent or more of water. Mixing and diluting hygiene product, with such a high percentage of water, enlarges their volume and causes massive pollution through their production and logistics as follows: mixing high amounts of water requires massive energy and machinery inputs in the production process for the mixing machines and conveying machinery, bottle filling machines, enormous storage spaces requirements, and internal logistics (forklifts, cranes, containers, shelves, etc.). Further to the direct environmentally harmful process, there are many highly pollutive processes involved with production and operation of equipment and machinery.

Almost all the efforts and inputs, involved with the product transportation, are actually devoted to transportation of water. The water handling effect includes a direct pollutive process is grossly inefficient transport of water for thousands of miles each time by highly pollutive vessels such as ships, trains, and trucks along with all collateral logistic equipment and containers. Additional indirect pollution is caused by the peripheral industries involved, such as trucks, ships and various equipment production industries. 95 percent and more of the environmental harming processes are dedicated to simply manipulation of water. The storage of water in warehouses and stores causes massive direct and indirect environmental harm. From giant electric power consuming warehouses built from pollutive construction materials, through logistic equipment such as cranes and forklifts to disposable packaging materials (stretch plastic, cardboard boxes, etc.) and fixed containing facilities (shelves, containers, etc.) packaging and. Therefore liquid soap, shampoo and most of all other hygiene and self-care products in use are harming the environment and contribute to unproportioned environmental abuse.

### SUMMARY OF THE INVENTION

The following summary of the invention is provided in order to provide a basic understanding of some aspects and features of the invention. This summary is not an extensive overview of the invention and as such it is not intended to particularly identify key or critical elements of the invention or to delineate the scope of the invention. Its sole purpose is to present some concepts of the invention in a simplified form as a prelude to the more detailed description that is presented below.

The invention was made in view of the deficiencies of the prior art and provides systems, methods and processes for overcoming these deficiencies. According to some embodiments and aspects of the present invention, there is provided an eco-friendly, healthy and economical care hygiene product, including a readily dissolvable water-soluble cartridge complex, containing all required active ingredients of the product. Therefore, instead of purchasing a bottle filled with the product, the user buys a solid readily dissolvable cartridge complex with a volume of only up to 20 percent of the ordinary product, puts the readily dissolvable cartridge complex in a reusable bottle, fills the bottle with water from the tap and in a short time receives a high-quality finished product, in a non-limiting manner including: shampoo, body soap, hand soap, conditioner, floor cleaner, dishwashing soap, home cleaning products, mouthwash and/or any other liquid with substantial water content.

The readily dissolvable cartridge complex, along with the savings of unnecessary water transportation, logistics and all the packaging material, provides an eco-friendly product, for less money than spent today on ordinary environmentally harming products.

In accordance with some aspects and embodiments of the present invention there is provided a readily dissolvable cartridge complex including: a segmental scaffolding including an effervescent material readily dissolvable in an aqueous solution; a plurality of radial partitions of the segmental scaffolding, arranged in tandem, extending about the longitudinal centerline of the dissolvable cartridge complex; a plurality of segments of the dissolvable cartridge complex, separated by the radial partitions and formed in-between the radial partitions; a plurality of axial partitions of the segmental scaffolding, extending transversally to and along the longitudinal centerline of the dissolvable cartridge complex, dividing each one of the plurality of the segments of the dissolvable cartridge complex into a plurality of sectors; a plurality of compartments formed in-between the axial partitions within the sectors, in each one of the plurality of the segments of the dissolvable cartridge complex; a plurality of structured payload pellets, accommodated in the compartments.

In some embodiments the dissolvable cartridge complex includes a coating configured to absorb and/or neutralize undesired additives or impurities in water.

In some embodiments the dissolvable cartridge complex includes a coating including a water softening agent. In some embodiments the dissolvable cartridge complex includes a coating including natrium chloride.

In accordance with some aspects and embodiments of the present invention there is provided an industrial process of fabricating a readily dissolvable cartridge complex including: preparing an effervescent material readily dissolvable in an aqueous solution; forming at least one segment of a segmental scaffolding from the effervescent material including: forming at least on radial partition of the at least one segment of the segmental scaffolding, extending about the longitudinal centerline of the dissolvable cartridge complex; forming a plurality of axial partitions within the at least one segment of the segmental scaffolding, extending transversally to and along the longitudinal centerline of the dissolvable cartridge complex; forming a plurality of compartments formed in-between the axial partitions, in each one of the plurality of the segments of the segmental scaffolding; preparing a payload substance; forming a plurality of structured payload substance pellets; accommodating the structured payload substance pellets in the compartments of the segmental scaffolding; compressing at least one segment of the segmental scaffolding of the readily dissolvable cartridge complex; iteratively forming a plurality of segments of the readily dissolvable cartridge complex separated by the radial partitions.

In some embodiments the industrial process further includes furnishing the readily dissolvable cartridge complex with a coating configured to absorb and/or neutralize undesired additives or impurities in water.

In some embodiments the industrial process further includes furnishing the readily dissolvable cartridge complex with a coating including water softening agent, including natrium chloride.

In some embodiments the industrial process further includes controllably saturating at least one member selected from the group consisting of: the payload substance and the effervescent material, with a predefined amount of saturating substrate, thereby conferring to the at least one member a semi-liquefied consistency.

In some embodiments the industrial process further includes deploying at least one stencil, in which an exterior surface of the at least one stencil essentially conforms and/or matches with at least one shape selected from the group consisting of: a shape of axial partitions of the segmental scaffolding, extending transversally to and along the longitudinal centerline of the dissolvable cartridge complex and a shape of the structured payload substance pellets.

In some embodiments the compressing of the industrial process further includes essentially conforming and/or matching with exterior outline of a shape of the structured payload substance pellets.

In accordance with some aspects and embodiments of the present invention there is provided an industrial manufacture system for fabricating a readily dissolvable cartridge complex includes: at least one form, including an essentially hollow shape, in which an interior surface of the at least one form essentially conforms and/or matches with a shape of the readily dissolvable cartridge complex; a segmental scaffolding fabricating machine including: an effervescent material reservoir, containing an effervescent material readily dissolvable in an aqueous solution; a dossing module connected to the reservoir of effervescent material, configured to receive the effervescent material from the reservoir of effervescent material and to portion a predetermined amount of the effervescent material; a fabricating module including at least one nozzle, configured to dispose the effervescent material in a predefined structured arrangement within the form, thereby forming a plurality of compartments, within at least one segment of the segmental scaffolding; a compacting mechanism, including at least one pressing piston operationally connected to a pressure exerting device, configured to compress at least one segment of the segmental scaffolding of the readily dissolvable cartridge complex within the form; a structured payload substance pellets fabricating machine including: a payload substance reservoir, containing a payload substance; a dossing module connected to the reservoir of payload substance, configured to receive the payload substance from the reservoir of payload substance and to portion a predetermined amount of the payload substance; a fabricating module including at least one nozzle, configured to dispose the payload substance in a predefined structured arrangement within the form, thereby forming a plurality of structured payload substance pellets within the compartments in at least one segment of the segmental scaffolding; a compacting mechanism, including at least one pressing piston operationally connected to a pressure exerting device, configured to compress the structured payload substance pellets within the compartments, thereby accommodating the structured payload substance pellets in the compartments, formed within at least one segment of the segmental scaffolding of the readily dissolvable cartridge complex, within the form.

In some embodiments the industrial manufacture system further includes a saturating module, configured for controllably saturate at least one member selected from the group consisting of: the payload substance and the effervescent material, with a predefined amount of saturating substrate, thereby conferring to the at least one member a semi-liquefied consistency.

In some embodiments the industrial manufacture system further includes a pulverizator, configured for furnishing the readily dissolvable cartridge complex with a coating configured to absorb and/or neutralize undesired additives or impurities in water.

In some embodiments the industrial manufacture system further includes at least one stencil, in which an exterior surface of the at least one stencil essentially conforms and/or matches with at least one shape selected from the group consisting of: a shape of axial partitions of the segmental scaffolding, extending transversally to and along the longitudinal centerline of the dissolvable cartridge complex; a shape of the structured payload substance pellets.

In some embodiments the compacting mechanism of the structured payload substance pellets fabricating machine includes a plurality of pressing pistons, in which an exterior outline of the pistons essentially conforms and/or matches with exterior outline of a shape of the structured payload substance pellets.

### DEFINITIONS

The term structured as referred to herein is to be construed as including any geometrical shape, exceeding in complexity a plain linear shape or a shape embodying simple cylindrical, elliptical or polygonal contour or profile. A more complex shape, a plain linear shape or a shape embodying simple cylindrical, elliptical or polygonal contour or profile, constitutes an example of structured geometry.

The term effervescent material as referred to herein is to be construed as including any material, mix of materials and/or composition capable of effervescence upon contact with water. Effervescence, exemplified in this context, can mean the emission of gas bubbles from a liquid as a result of a chemical reaction between a soluble acid source and an alkali metal carbonate to produce carbon dioxide gas.

The term payload substance as referred to herein is to be construed as including any material, mix of materials and/or composition including an anhydrous or essentially dry concentrated form and/or concentrate of any hygiene products, such as liquid soaps, body wash, hair care products and any other cosmetics, as well as any other household and/or hygiene products. The term payload substance as referred to herein can be construed as including any active ingredient of any household and/or hygiene product.

The terms method and process as used herein are to be construed as including any sequence of steps or constituent actions, regardless a specific timeline for the performance thereof. The particular steps or constituent actions of any given method or process are not necessarily in the order they are presented in the claims, description or flowcharts in the drawings, unless the context clearly dictates otherwise. Any particular step or constituent action included in a given method or process may precede or follow any other particular step or constituent action in such method or process, unless the context clearly dictates otherwise. Any particular step or constituent action and/or a combination thereof in any method or process may be performed iteratively, before or after any other particular step or action in such method or process, unless the context clearly dictates otherwise. Moreover, some steps or constituent actions and/or a combination thereof may be combined, performed together, performed concomitantly and/or simultaneously and/or in parallel, unless the context clearly dictates otherwise. Moreover, some steps or constituent actions and/or a combination thereof in any given method or process may be skipped, omitted, spared and/or opted out, unless the context clearly dictates otherwise.

The term modular, as referred to herein, should be construed as a stand-alone unit. The term modular *inter alia* means a standardized unit that may be conveniently installed or deployed without significant impact to the environment. The term modular, however, doesn't necessarily mean providing for ease of interchange or replacement. The term modular is optionally satisfied by providing for ease of at least onetime deployment or installation.

The term readily connectable, as referred to herein, should be construed as a standardized unit that may be conveniently connected to other components of the system. The term readily connectable, however, doesn't necessarily mean readily disconnectable or removable. The term readily connectable is optionally satisfied by providing for ease of at least onetime connection or coupling.

By operationally connected and operably coupled or similar terms used herein is meant connected in a specific way (e.g., in a manner allowing fluid to move and/or electric power to be transmitted) that allows the disclosed system and its various components to operate effectively in the manner described herein.

The term fluid or liquid as referred to herein is to be construed as any material that deforms when a shear stress is applied. While fluid generally would refer to any liquids or gases, it may be used herein to describe fluidized solids and bulk solids and/or granulate matter that are capable of flowing or otherwise moving inside a device as a result of pressure differences and/or gravitational force. Such materials may include slurries, suspensions, pastes, powders, granular solids, particle solids, granulate matter, particulate matter, as well as any combinations thereof.

The term slurry, as referred to herein, is to be construed as a mixture of solids denser than water suspended in liquid, usually water. Solids concentrations in a slurry typically range between about 0.5 percent and about 5 percent.

The term sludge, as referred to herein, is to be construed as a semi-solid slurry. The term is also sometimes used as a generic term denoting solids separated from suspension in a liquid. Solids concentrations in a sludge typically range between about 5 percent and about 15 percent.

In the specification or claims herein, any term signifying an action or operation, such as: a verb, whether in base form or any tense, gerund or present/past participle, is not to be construed as necessarily to be actually performed but rather in a constructive manner, namely as to be performed merely optionally or potentially.

The term substantially as used herein is a broad term, and is to be given its ordinary and customary meaning to a person of ordinary skill in the art (and is not to be limited to a special or customized meaning), and refers without limitation to being largely but not necessarily entirely of that quantity or quality which is specified.

The term essentially means that the composition, method or structure may include additional ingredients, stages and or parts, but only if the additional ingredients, the stages and/or the parts do not materially alter the basic and new characteristics of the composition, method or structure claimed.

As used herein, the term essentially changes a specific meaning, meaning an interval of plus or minus ten percent (± 10 percent). For any embodiments disclosed herein, any disclosure of a particular value, in some alternative embodiments, is to be understood as disclosing an interval approximately or about equal to that particular value (i.e., ± 10 percent).

As used herein, the terms about or approximately modify a particular value, by referring to a range equal to the particular value, plus or minus twenty percent (+/-20 percent). For any of the embodiments, disclosed herein, any disclosure of a particular value, can, in various alternate embodiments, also be understood as a disclosure of a range equal to about that particular value (i.e. +/-20 percent).

As used herein, the term or is an inclusive or operator, equivalent to the term and/or, unless the context clearly dictates otherwise; whereas the term and as used herein is also the alternative operator equivalent to the term and/or, unless the context clearly dictates otherwise.

It should be understood, however, that neither the briefly synopsized summary nor particular definitions hereinabove are not to limit interpretation of the invention to the specific forms and examples but rather on the contrary are to cover all modifications, equivalents and alternatives falling within the scope of the invention.

### DESCRIPTION OF THE DRAWINGS

The present invention will be understood and appreciated more comprehensively from the following detailed description taken in conjunction with the appended drawings in which:
**FIG 1A** is a perspective view of a readily dissolvable cartridge complex, according to some embodiments of the present invention;
**FIG 1B** is an enlarged perspective view of the readily dissolvable cartridge complex, according to some embodiments of the present invention;
**FIG 2A** is a top view of a segment of the readily dissolvable cartridge complex, according to some embodiments of the present invention;
**FIG 2B** is a perspective view of a segment of the readily dissolvable cartridge complex, according to some embodiments of the present invention;
**FIG 3A** is an isometric view of a readily dissolvable cartridge complex, according to some embodiments of the present invention;
**FIG 3B** is a top view of a segment of the readily dissolvable cartridge complex, according to some embodiments of the present invention;
**FIG 3C** is a perspective view of a compartment of a segment of the readily dissolvable cartridge complex, according to some embodiments of the present invention;
**FIG 4A** is a perspective view of a reusable dispenser bottle into which a readily dissolvable cartridge complex is introduced, according to some embodiments of the present invention;
**FIG 4B** is an exploded view of a reusable dispenser bottle into which dissolvable cartridge complex is introduced, according to some embodiments of the present invention;
**FIG 4C** is an isometric view of plurality of cups of plurality of reusable dispenser bottles in a nested and stacked configuration, according to some embodiments of the present invention;
**FIG 4D** is a side view of a cover of a reusable dispenser bottle, according to some embodiments of the present invention;
**FIG 4E** is a top view of a cover of a reusable dispenser bottle, in a closed configuration, according to some embodiments of the present invention;
**FIG 4F** is a top view of a cover of a reusable dispenser bottle, in an open configuration, according to some embodiments of the present invention;
**FIG 5** is a block diagram of an industrial manufacture system for fabricating a readily dissolvable cartridge complex, according to some embodiments of the present invention;
**FIG 6A** is an exploded view of a form and compacting mechanism complex including a stencil of an industrial manufacture system for dry fabricating of a readily dissolvable cartridge complex, according to some embodiments of the present invention;
**FIG 6B** is a schematic view of a form and compacting mechanism complex including a stencil of an industrial manufacture system for dry fabricating of a readily dissolvable cartridge complex, according to some embodiments of the present invention, showing the stencil within the form;
**FIG 7A** is an exploded view of a form and compacting mechanism complex including a structured nozzle of an industrial manufacture system for fabricating of a readily dissolvable cartridge complex, according to some other embodiments of the present invention;
**FIG 7B** is a schematic view of a structured nozzle introduced into the form, according to some other embodiments of the present invention;
**FIG 7C** is a schematic view of the formation of one segment of the readily dissolvable cartridge complex, according to some other embodiments of the present invention;
**FIG 7D** is a schematic view of the formation of a radial partition of the readily dissolvable cartridge complex, according to some other embodiments of the present invention;
**FIG 7E** is a schematic view of the readily dissolvable cartridge complex formed within the form, according to other embodiments of the present invention;
**FIG 7F** is an exploded view of the readily dissolvable cartridge complex formed within the form and a pressing array, according to some other embodiments of the present invention;
**FIG 7G** is a schematic view of a pressing piston compacting the readily dissolvable cartridge complex within the form, according to some other embodiments of the present invention;
**FIG 7H** is a schematic view of a pressing piston ejecting the readily dissolvable cartridge complex from the form, according to some other embodiments of the present invention;
**FIG 7I** is a schematic view of a pressing piston withdrawn into the from after the readily dissolvable cartridge complex was ejected from, according to some other embodiments of the present invention;
**FIG 8** is a flowchart of an industrial process of fabricating a readily dissolvable cartridge, according to some embodiments of the present invention;
**FIG 9** is a flowchart of an industrial process of essentially dry fabricating a readily dissolvable cartridge, according to some embodiments of the present invention;
**FIG 10** is a flowchart of an industrial process of essentially dry fabricating a readily dissolvable cartridge, according to some other embodiments of the present invention.

While the invention is susceptible to various modifications and alternative forms, specific embodiments thereof have been shown merely by way of example in the drawings. The drawings are not necessarily complete and components are not essentially to scale; emphasis instead being placed upon clearly illustrating the principles underlying the present invention.

### DETAILED DISCLOSURE OF EMBODIMENTS

Illustrative embodiments of the invention are described below. In the interest of clarity, not all features of actual implementation are described in this specification. It should be appreciated that various features or elements described in the context of some embodiment may be interchangeable with features or elements of any other embodiment described in the specification. Moreover, it will be appreciated that for the development of any actual embodiment, numerous implementation-specific decisions must be made to achieve the developers' specific goals, such as compliance with technology- or business-related constraints, which may vary from one implementation to another, and the effort of such a development might be complex and time-consuming, but would nevertheless be a routine undertaking for those of ordinary skill in the art having the benefit of this disclosure.

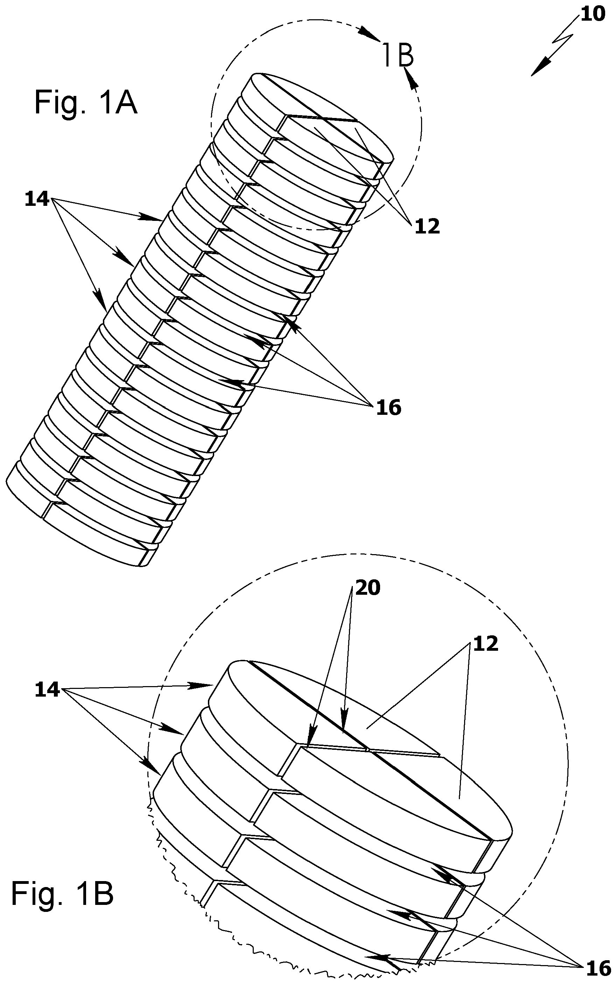

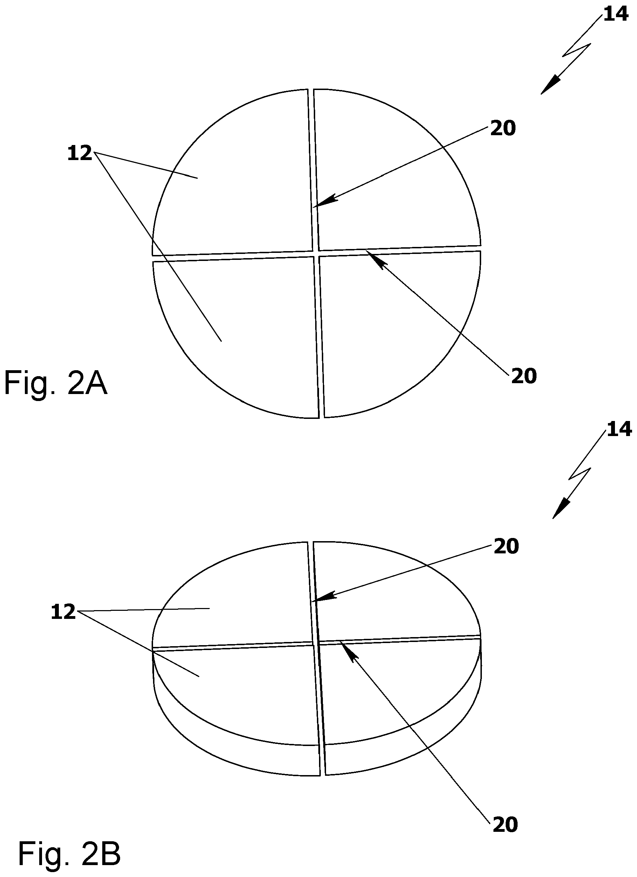

In accordance with some embodiments of the present invention, reference is now made to **FIG 1A** to **3C****,** showing readily dissolvable cartridge complex **10.** In some embodiments, readily dissolvable cartridge complex **10** comprises a plurality of structured payload substance pellets **12,** embedded within individual segments **14** of a segmental scaffolding. Segments **14** of segmental scaffolding comprises an effervescent material readily dissolvable in an aqueous solution.

The effervescent material of the segmental scaffolding may be selected from appropriate material which exhibits effervesce on contact with water. In one example, the effervescent material comprises at least sodium bicarbonate and citric acid. These materials are present in any suitable amounts to achieve effervescence. One skilled in the art is able to combine these materials to provide the desired rate of effervescence.

In other examples, the effervescent material comprises sodium bicarbonate in an amount of 40 to 75 wt percent. In other examples, the effervescent material comprises sodium bicarbonate in an amount of 50 to 70 wt percent. In other examples, the effervescent material comprises sodium bicarbonate in an amount of 55 to 70 wt percent. In some examples, the effervescent material comprises citric acid in an amount of 24 to 40 wt percent. In other examples, the effervescent material comprises citric acid in an amount of 26 to 38 wt percent based on the first effervescent material. In other examples, the effervescent material comprises citric acid in an amount of 28 to 36 wt percent. In other examples, the effervescent material comprises citric acid in an amount of 30 to 34 wt percent. In some examples, the effervescent material comprises sodium bicarbonate in an amount of 40 to 75 wt percent and citric acid in an amount of 24 to 40 wt percent.

In some embodiments, pellets **12** comprise a payload substance, including a surfactant in an amount of 0.5 to 5 wt percent. The surfactant of the payload substance is primarily selected from those surfactants known in the art to be suitable for contact with the skin. In one embodiment, the surfactant is selected from the group consisting of sodium laureth sulfate, cocamide diethanolamine, lauryl betaine and mixtures thereof. In one embodiment, the surfactant is sodium laureth sulfate.

In some embodiments, the surfactant of the payload substance provides the composition with the ability to achieve its required purpose. Thus for a cleanser, the surfactant creates foam and removes dirt and grease from the user's skin.

In some embodiments, payload substance pellets **12** further comprise a fragrance. Preferably the fragrance is present in an amount of 0.5 to 4 wt percent. In some embodiments, payload substance pellets **12** comprise fragrance in an amount of 1 to 4 wt percent. In some embodiments, payload substance pellets **12** comprise fragrance in an amount of 2 to 4 wt percent. In some embodiments, payload substance pellets **12** comprise fragrance in an amount of 2.5 to 3.5 wt percent.

In some embodiments, payload substance pellets **12** further comprise another material, whether soluble or insoluble, which us incorporated into the product to provide further effects, for example with regard to odor, taste, pH, texture colour or foam production and etc.

In some embodiments, readily dissolvable cartridge complex **10** further comprises a plurality of radial partitions **16** of segmental scaffolding. Radial partitions **16** are arranged in tandem, extending about a longitudinal centerline of dissolvable cartridge complex **10.** Radial partitions **16** divide adjacent segments **14** of the segmental scaffolding. In some embodiments, readily dissolvable cartridge complex **10** comprises plurality of segments **14.** Segments **14** are separated by radial partitions **16** and formed in-between radial partitions **16.**

In some embodiments, readily dissolvable cartridge complex **10** comprises plurality of axial partitions **20** of the segmental scaffolding. Axial partitions **20** extend transversally to and along the longitudinal centerline of dissolvable cartridge complex **10,** dividing each one of plurality of segments **14.**

In some embodiments, the segmental scaffolding of readily dissolvable cartridge complex **10** further comprises a plurality of compartments. The compartments are formed in-between axial partitions **20** of the segmental scaffolding, in each one of plurality of segments **14** of the segmental scaffolding. In some embodiments, readily dissolvable cartridge complex **10** comprises a plurality of structured payload pellets **12.** Structured payload pellets **12** are accommodated in the compartments of the segmental scaffolding.

In some embodiments, dissolvable cartridge complex **10** is configured for rapidly or even quote instantly providing for an extensive surface area of structured payload pellets **12,** upon contact with water. After the segmental scaffolding undergoes rapid or even quote instant effervescence, the accumulative surplus surface area of all structured payload pellets **12** accelerates the solubility and increasing mixing and molecule diffusion rate, by removing radial partitions **16** and axial partitions **20** of the segmental scaffolding.

In some embodiments, dissolvable cartridge complex **10** is coated by dissoluble coating **24.** When dissolvable cartridge complex **10** is placed in water or other essentially aqueous solution, dissoluble coating **24** rapidly or even quote dissolves in water. Then uncoated dissolvable cartridge complex **10** is exposed to water thereby initiating an effervescence reaction. Dissoluble coating **24** is configured to modify the water or other aqueous solution, so as to sequester, absorb, precipitate, balance or otherwise neutralize undesired impurities or additives in the water.

In some examples, dissoluble coating **24** comprising a water softening agent, such as natrium chloride, configured to neutralize water hardening additives in the water, such as calcium and magnesium ions. In other examples, dissoluble coating **24** comprising a sequestering agent, such as EDTA or other chelate forming agent, configured to sequester metal ions in aqueous solution. In yet other examples, dissoluble coating **24** comprising a disinfectant, such as an oxidating or bleaching agent, configured to neutralize biological impurities in the water, for instance: iodine, chlorine, chloramine, chlorine and dioxide, and/or malodor treatment.

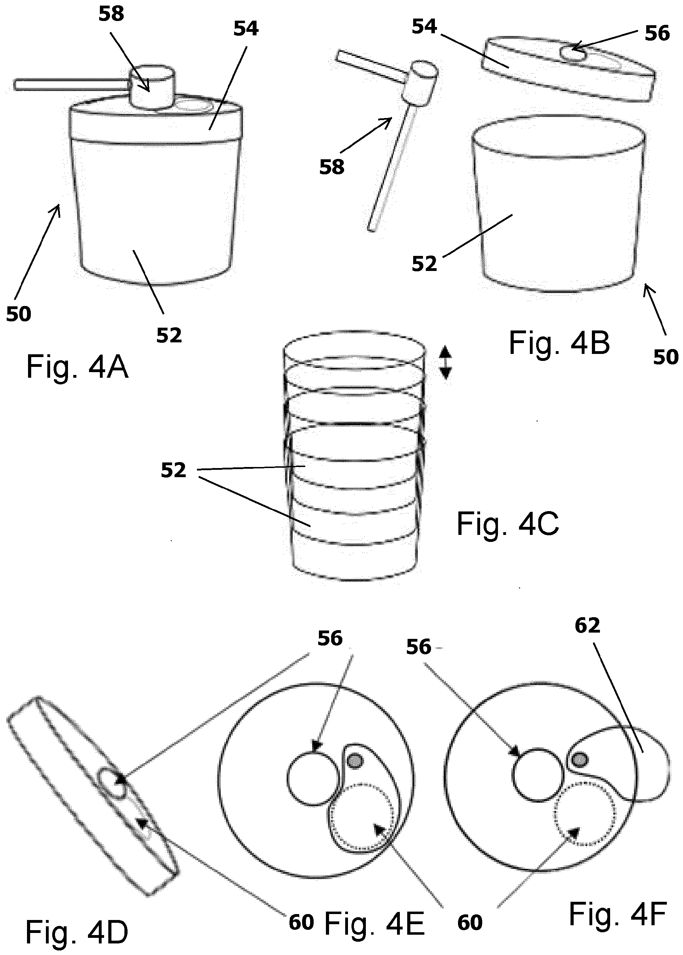

In accordance with some embodiments of the present invention, reference is now made **FIG 4A to 4F** showing reusable dispenser **50** into which dissolvable cartridge complex **10,** shown in **FIG 1A** to **3C** and described hereinabove, is administrated. The reusable dispenser of the embodiment of **FIG 4A** to **4F** illustrates various features that may be interchangeable with elements of any other embodiment described in the specification.

In some embodiments, reusable dispenser **50** comprises container **52.** Container **52** is configured for receiving the dissolvable cartridge complex, such as dissolvable cartridge complex **10** shown in **FIG 1A** to **3C****.** In some embodiments, container **52** is covered by cover **54.** Cover **54** preferably comprises aperture **56.** Aperture **56** is typically configured for accommodating dispensing pump **58.**

In some examples, dispensing pump **58** is of the type which is pressed with manual force to dispense the liquid. In other examples, dispensing pump **58** is of the electromechanical type, coupled to a power source and driven by electric power, to perform the dispensing action. In some examples, electromechanical dispensing pump **58** is manually activated upon physical contact with an electric switch, knob or button, whereas in other examples electromechanical dispensing pump **58** is configured for contactless activation, typically by an IR detector. In some examples, dispensing pump **58** comprises a foam dispenser, comprising a mechanism configured to transform the liquid dispensed from reusable dispenser **50** into a light or thick foam.

In some embodiments, cover **54** of reusable dispenser **50** further comprises cartridge aperture **60.** The dissolvable cartridge complex, such as dissolvable cartridge complex **10** shown in **FIG 1A** to **3C****,** is introduced into container **52** through cartridge aperture **60.** Cartridge aperture **60** is optionally coverable by lid **62.** Preferably, lid **62** is operationally connected to cover **54.** It should be acknowledged that the configuration of reusable dispenser **50** with cover **54,** with cover on top, with cartridge aperture **60** is merely exemplary, whereas any other configuration is equally applicable.

In some embodiments reusable dispenser bottle **50** is eco-efficient and reusable as well as recyclable. In some examples, reusable dispenser bottle **50** is made of stainless steel and/or recycled aluminum and/or any other material or composition. In other embodiments, reusable dispenser bottle **50** is disassembled so that a plurality of containers **52** and plurality of covers **54** are nested in a stack one on top of another, such as shown in **FIG 4C****,** thereby reducing shipping volume of dispensers **50.**

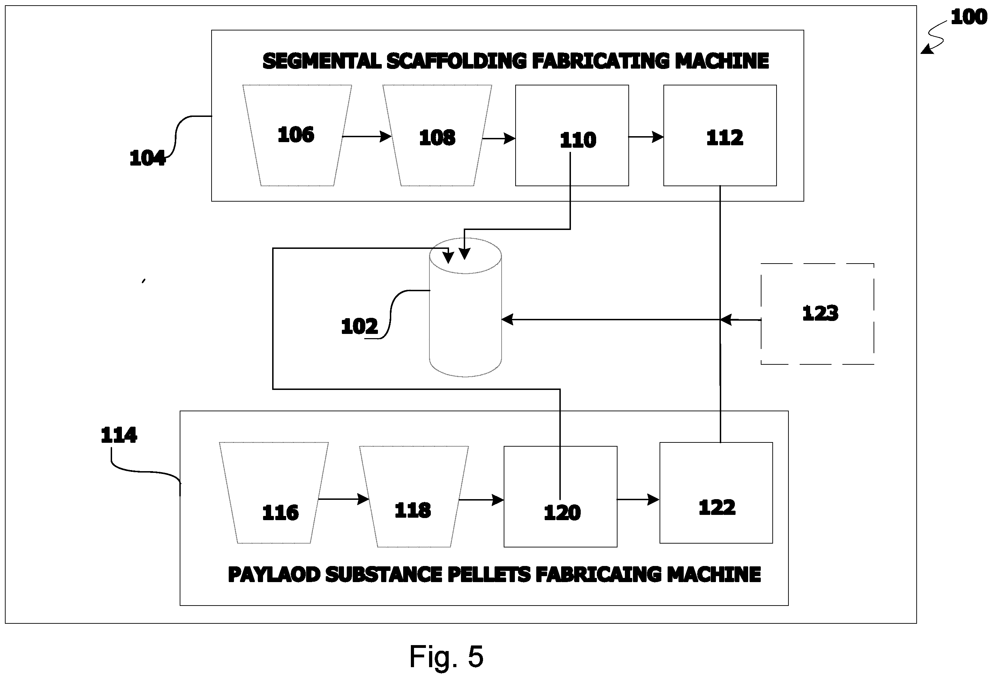

In accordance with some embodiments of the present invention, reference is now made to **FIG 5****,** showing industrial manufacture system **100** for fabricating a readily dissolvable cartridge complex. Industrial manufacture system **100** of the embodiment of **FIG 5** illustrates various features that may be interchangeable with elements of any other embodiment described in the specification.

In some embodiments, system **100** for fabricating a readily dissolvable cartridge complex comprises at least one form **102.** Form **102** comprising an essentially hollow shape, in which an interior surface of at least one form **102** essentially conforms and/or matches with a shape of dissolvable cartridge complex, such as dissolvable cartridge complex **10** shown in **FIG 1A** to **3C** and described hereinabove.

The system **100** further comprises segmental scaffolding fabricating machine **104.** The segmental scaffolding fabricating machine **104** comprises effervescent material reservoir **106.** Effervescent material reservoir **106** contains and stores an effervescent material readily dissolvable in an aqueous solution. In some examples, effervescent material reservoir **106** comprises a conical stainless steel reservoir or a miniature silo. In some embodiments, the effervescent material effervescent material in effervescent material reservoir **106** is typically in the form of dry powder. The term dry powder, as referred to herein, may include any substance comprising one or a plurality of constituents or ingredients with one or a plurality of (average) particulate size ranges.

The segmental scaffolding fabricating machine **104** comprises dosing module **108.** Dosing module **108** is connected to effervescent material reservoir **106** and configured to receive the effervescent material from effervescent material reservoir **106.** Dosing module **108** is configured to portion a predetermined amount of the effervescent material.

The dosing module **108** comprises a predefined volume and/or
a weighting scale device. Dosing module **108** is configured to portion the effervescent material of various predetermined amounts, volumes and or weights. From many kilograms and bulk volume of effervescent material in reservoir **106,** dosing module **108** portions relatively small and predetermined amounts.

The segmental scaffolding fabricating machine **104** comprises fabricating module **110.** Fabricating module **110** comprises at least one nozzle, configured to dispose the effervescent material in a predefined structured arrangement within form **102,** thereby forming a plurality of compartments, within at least one segment of the segmental scaffolding. The fabricating module **110** comprises at least one nozzle, configured to dispose the effervescent material in a predefined location and/or coordinate within form **102** in a controllable manner, somewhat similarly to a jet printer, thereby gradually forming the radial and axial portions step-by-step, of at least one segment of the segmental scaffolding.

The segmental scaffolding fabricating machine **104** comprises compacting mechanism **112.** Compacting mechanism **112** comprises at least one pressing piston operationally connected to pressure exerting device **123,** configured to compress at least one segment of the readily dissolvable cartridge complex within form **102.** In some embodiments, when the effervescent material is compressed, it undergoes agglomeration and is capable thereafter to inherently maintain the structural integrity of the readily dissolvable cartridge complex.

In some embodiments, system **100** is configured for semi-liquefied fabricating of the readily dissolvable cartridge complex. In such embodiments, segmental scaffolding fabricating machine **104** of system **100** further comprises a saturating module, configured for controllably saturating the effervescent material, with a predefined amount of saturating substrate, thereby conferring to the effervescent material a semi-liquefied consistency. In some examples, the saturating module comprises a substrate storage unit and/or a substrate dosimeter. The substrate storage unit is configured storing the substrate or liquid added to the effervescent material, whereas the substrate dosimeter is configured for controllably adding a certain amount of substrate or liquid to the effervescent material, thereby conferring to the effervescent material a semi-liquefied consistency.

In some embodiments, segmental scaffolding fabricating machine **104** comprises a blender. The blender is configured for mixing the dosed saturating substrate or liquid with a predefined amount of effervescent material, thereby conferring homogeneity to the effervescent material, optionally with semi-liquefied consistency.

The system **100** further comprises structured payload substance pellets fabricating machine **114.** The In some embodiments, structured payload substance pellets fabricating machine **114** comprises payload substance reservoir **116.** In some embodiments, the payload substance in payload substance reservoir **116** is typically in the form of dry powder. Payload substance reservoir **116** contains and stores a payload substance. In some examples, payload substance reservoir **116** comprises a conical stainless steel reservoir or a miniature silo.

The structured payload substance pellets fabricating machine **114** further comprises dosing module **118.** Dosing module **118** is connected to payload substance reservoir **116** and configured to receive the payload substance from reservoir **116.** Dosing module **118** is configured to portion a predetermined amount of the payload substance. Dosing module **108** is configured to portion a predetermined amount of the payload substance.

The structured payload substance pellets fabricating machine **114** comprises fabricating module **120.** The payload substance pellets fabricating module **120** comprises at least one nozzle, configured to dispose the payload substance, optionally having a semi-liquefied consistency. The at least one nozzle is configured to dispose the payload substance in a predefined structured arrangement within form **102,** thereby forming a plurality of structured payload substance pellets within the compartments of the segmental scaffolding, in at least one segment of the of the readily dissolvable cartridge complex. In some preferred embodiments, fabricating module **120** comprises at least one nozzle, configured to dispose the payload substance in a predefined location and/or coordinate within form **102** in a controllable manner, somewhat similarly to a jet printer, thereby gradually forming the structured payload substance pellets step-by-step, of at least one segment of the readily dissolvable cartridge complex.

In some embodiments, the at least one nozzle of fabricating module **110** configured to dispose the effervescent material in a predefined location and/or coordinate within form **102,** as well as the at least one nozzle of fabricating module **120** configured to dispose the payload substance in a predefined location and/or coordinate within form **102,** in a controllable manner somewhat similarly to a jet printer, thereby gradually forming step-by-step the segment of the segmental scaffolding, are exchangeable nozzles on a same disposing machine, such as an extruder, dispenser, press, pump or ejector.

Fabricating module **110** comprises at least one nozzle, configured to dispose the effervescent material in a predefined structured arrangement within form **102,** thereby forming a plurality of compartments, within at least one segment of the segmental scaffolding. In some preferred embodiments, fabricating module **110** comprises at least one nozzle, configured to dispose the effervescent material in a predefined location and/or coordinate within form **102** in a controllable manner, somewhat similarly to a jet printer, thereby gradually forming the radial and axial portions step-by-step, of at least one segment of the segmental scaffolding.

In some preferred embodiments, fabricating module **120** comprises at least one structured nozzle, for instance embodying the shape of the segmental scaffolding and/or of the structured payload substance pellets and/or any portion thereof. A structured nozzle optionally disposes an entire fragment of at least one segment of the readily dissolvable cartridge complex, at once.

The structured payload substance pellets fabricating machine **114** further comprises compacting mechanism **122.** Compacting mechanism **122** comprises at least one pressing piston operationally connected to pressure exerting device **123,** configured to compress the structured payload substance pellets within the compartments of the segmental scaffolding, thereby accommodating the structured payload substance pellets in the compartments, formed within at least one segment of the segmental scaffolding of the readily dissolvable cartridge complex, within form **102.**

In some embodiments, system **100** is configured for semi-liquefied fabricating of the readily dissolvable cartridge complex. In such embodiments, structured payload substance pellets fabricating machine **114** of system **100** further comprises a saturating module, configured for controllably saturating the payload substance, with a predefined amount of saturating substrate, thereby conferring to the payload substance a semi-liquefied consistency. In some examples, the saturating module comprises a substrate storage unit and/or a substrate dosimeter. The substrate storage unit is configured storing the substrate or liquid added to the payload substance, whereas the substrate dosimeter is configured for controllably adding a certain amount of substrate or liquid to the payload substance, thereby conferring to the payload substance a semi-liquefied consistency.

In some embodiments, structured payload substance pellets fabricating machine **114** comprises a blender. The blender is configured for mixing the dosed saturating substrate or liquid with a predefined amount of payload substance, thereby conferring homogeneity to the payload substance with semi-liquefied consistency.

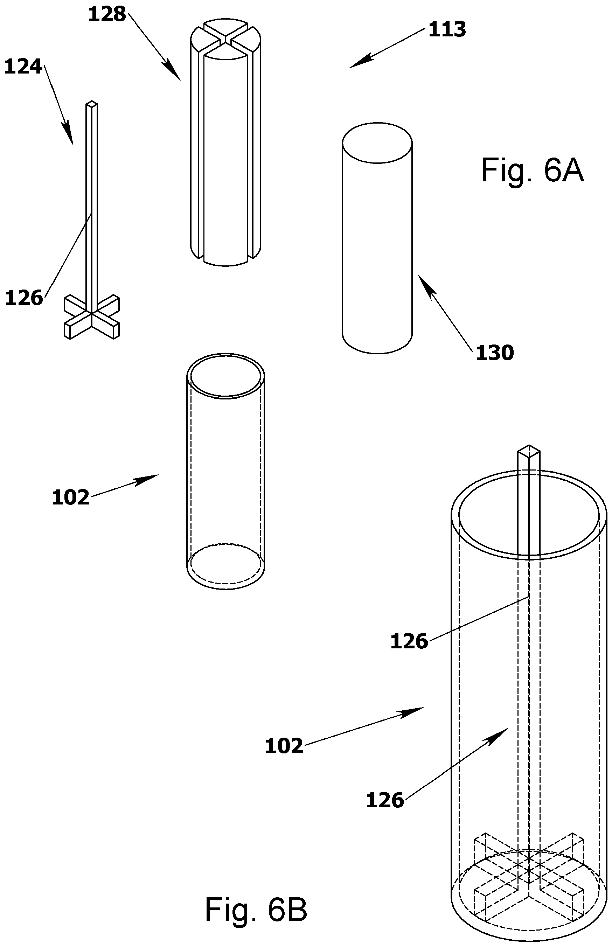

Reference is now made to **FIG 6A** and **6B****,** showing an example of assembly **113,** including the constituents of form **102,** fabricating module **110** and compacting module **112** of the segmental scaffolding fabricating machine **104** and fabricating module **120** and compacting module **122** of the structured payload substance pellets fabricating machine **114** of the industrial manufacture system **100** shown in **FIG 5****,** configured for essentially dry fabricating of a readily dissolvable cartridge complex. The example of assembly **113** of the embodiment of **FIG 6A** and **6B** illustrates various features that may be interchangeable with elements of any other embodiment described in the specification.

In some embodiments, the industrial manufacture system comprises at least one stencil **124.** In some embodiments, the exterior outline of least one stencil **124** essentially conforms and/or matches with a shape of axial partitions of the segmental scaffolding, extending transversally to and along the longitudinal centerline of the dissolvable cartridge complex. In some embodiments, at least one stencil **124** comprises mandrel **126.** Mandrel **126** is configured for manipulating the distal part of stencil **124** within form **102.**

In some embodiments, compacting mechanism **112** comprises pellet pressing piston array **128.** The exterior outline of the pistons, in pellet pressing piston **128,** essentially conforms and/or matches with exterior outline of a shape of the structured payload substance pellets. Pellet pressing piston array **128** is operationally connected to pressure exerting device **123,** configured to compress the structured payload substance pellets, within form **102.**

In some embodiment, compacting mechanism **112** further comprises segment pressing piston **130** operationally connected to pressure exerting device **123.** Segment pressing piston **130** configured to compress at least one segment of the segmental scaffolding of the readily dissolvable cartridge complex within form **102.**

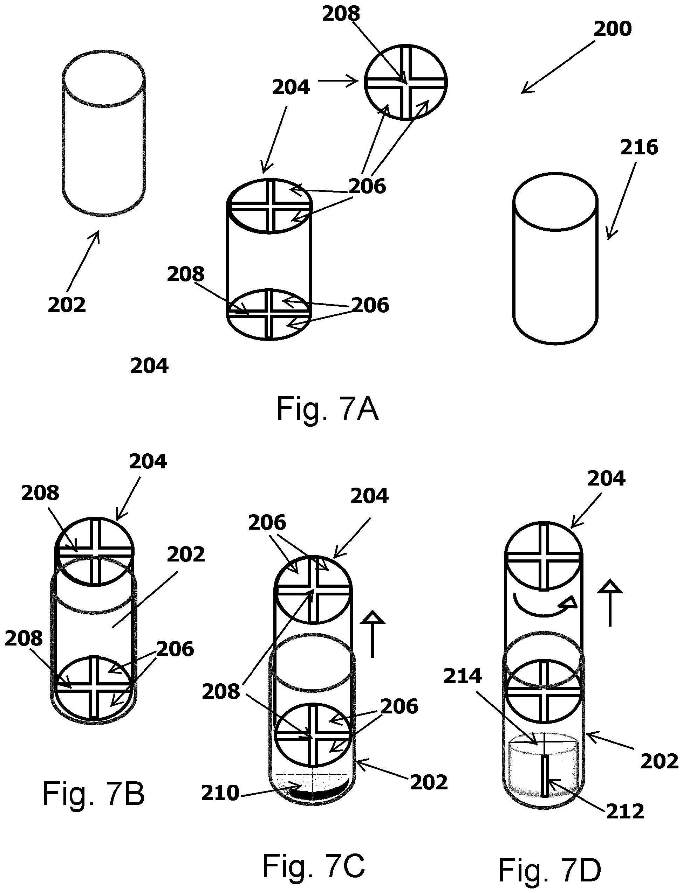

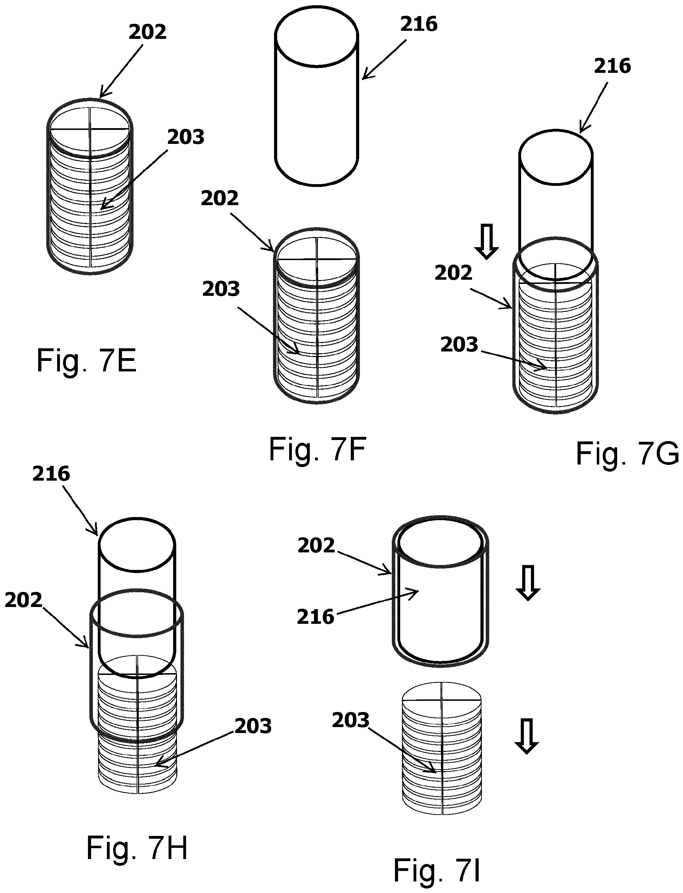

Reference is now made to **FIG 7A** to **7I****,** showing another example of assembly 200, including the constituents of form **102,** fabricating module **110** and compacting module **112** of the segmental scaffolding fabricating machine **104** and fabricating module **120** and compacting module **122** of the structured payload substance pellets fabricating machine **114** of the industrial manufacture system **100** shown in **FIG 5****,** configured for essentially dry fabricating of a readily dissolvable cartridge complex. The example of assembly **200** of the embodiment of **FIG 7A** to **7I** illustrates various features that may be interchangeable with elements of any other embodiment described in the specification.

In some embodiments, assembly **200** comprises form **202.** Form **202** comprising an essentially hollow shape, in which an interior surface of form **202** essentially conforms and/or matches with a shape of dissolvable cartridge complex **203,** such as dissolvable cartridge complex **10** shown in **FIG 1A** to **3C** and described hereinabove. It should be appreciated that the shape of the dissolvable cartridge complex, such as complex **10** shown in **FIG 1A** to **3C** and/or complex **203,** is shown as cylindrical merely by a way of example, whereas numerous profiled shapes, such as rectangular, elliptical, quadratic, triangular, polygonal and/or structured are equally applicable.

In some embodiments, assembly **200** further comprises structured nozzle **204.** The exterior outline of structured nozzle **204** essentially conforms and/or matches with exterior outline of dissolvable cartridge complex **203,** whereas the shape of the interior outline of structured nozzle **204** essentially conforms and/or matches with exterior outline the structured payload substance pellets and with a shape of axial partitions of the segmental scaffolding, extending transversally to and along the longitudinal centerline of dissolvable cartridge complex **203.**

In some embodiments, structured nozzle **204** comprises a plurality of payload substance nozzles **206.** Payload substance nozzles **206** are configured for administrating the payload substance into form **202,** thereby forming a plurality of structured payload substance pellets. In some embodiments, structured nozzle **204** comprises effervescent material nozzle **208.** Effervescent material nozzle **208** is configured for administrating the effervescent material into form **202,** thereby forming the axial partitions between the pellets, as well as the radial partitions between the segments.

In some embodiments, structured nozzle **204** with plurality payload substance nozzles **206** and effervescent material nozzle **208** is insertable into form **202.** Payload substance nozzles **206** and effervescent material nozzle **208** are implementable for administrating, optimally concomitantly, the payload substance and the effervescent material, while vertically translating structured nozzle **204** within form **202** in an upward direction, thereby forming first segment **210** of dissolvable cartridge complex **203** within form **202.**

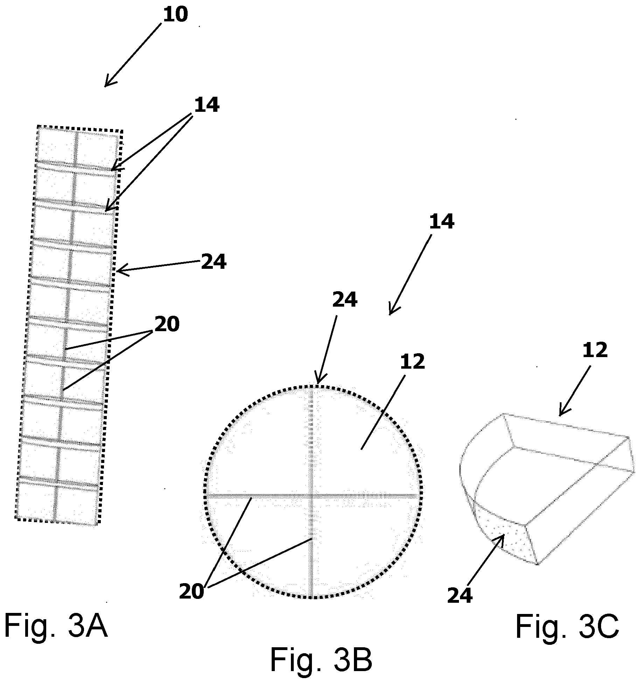

In some embodiments, structured nozzle **204** is rotatable about longitudinal centerline of structured nozzle **204** while additionally filling from effervescent material nozzle **208** a layer of effervescent material above constructed segment **210,** thereby providing material for radial partition **212,** such as radial partitions **16** shown in **FIG 3A****,** between the segments of dissolvable cartridge complex **203.**

In some embodiments, upon administering the effervescent material for radial partition **214,** payload substance nozzles **206** and effervescent material nozzle **208** are implementable for administrating, optimally concomitantly, the payload substance and the effervescent material, while vertically translating structured nozzle **204** within form **202** in an upward direction, thereby forming the next segment of dissolvable cartridge complex **203.**

In some embodiments, assembly **200** further comprises pressing piston **216.** Pressing piston **216** is insertable into form **203** once structured nozzle **204** is withdrawn therefrom. The exterior outline of pressing piston **216,** essentially conforms and/or matches with exterior outline of a shape of the readily dissolvable cartridge complex. Pressing piston **210** is configured to compact readily dissolvable cartridge complex **203** within form **202** and/or for ejecting readily dissolvable cartridge complex **203** from form **202.**

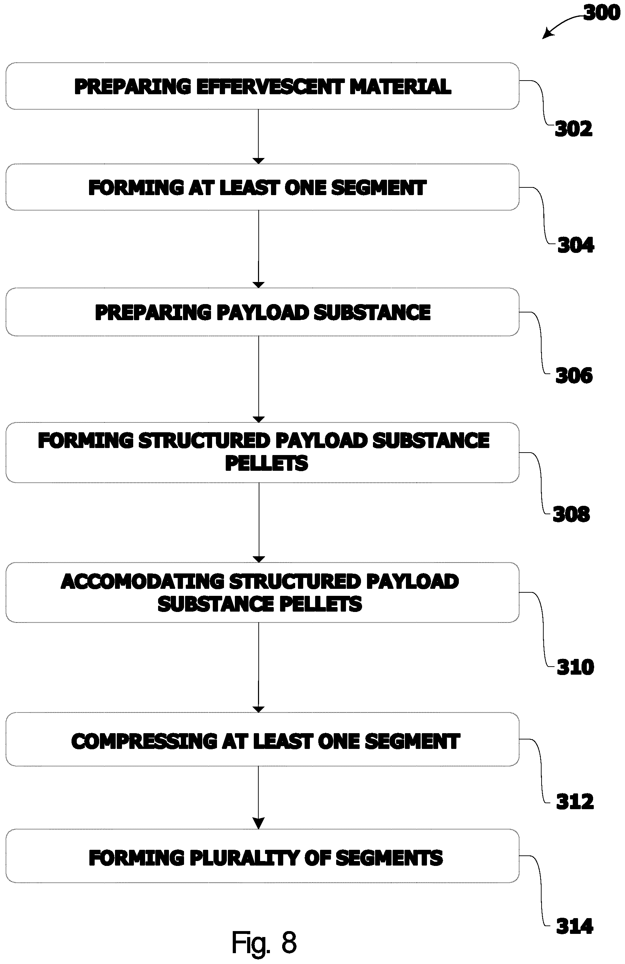

In accordance with some embodiments of the present invention, reference is now made **FIG 8** showing a flowchart of industrial process **300** of fabricating a readily dissolvable cartridge complex. The process of the embodiment of **FIG 8** illustrates various features that may be interchangeable with elements of any other embodiment described in the specification.

The process **300** commences with step **302** of preparing an effervescent material readily dissolvable in an aqueous solution. The process **300** comprises step **304** of forming at least one segment of a segmental scaffolding from the effervescent material. Step **304** typically further comprises forming at least on radial partition of at least one segment of the segmental scaffolding, extending about the longitudinal centerline of the dissolvable cartridge complex.

The step **304** comprises forming a plurality of axial partitions within at least one segment of the segmental scaffolding, extending transversally to and along the longitudinal centerline of the dissolvable cartridge complex. The step **304** further comprises forming a plurality of compartments formed in-between the axial partitions, in each one from the plurality of the segments of the segmental scaffolding.

The process **300** includes step **306** of preparing a payload substance. In some embodiments, process **300** further comprises step **308** of forming a plurality of structured payload substance pellets. The step **304** of forming at least one segment of a segmental scaffolding from the effervescent material is performed concomitantly with step **308** of forming a plurality of structured payload substance pellets, by a plurality of nozzles.

The process **300** includes step **310** of accommodating the structured payload substance pellets in the compartments of the segmental scaffolding. The process **300** further comprises step **312** of compressing at least one segment of the segmental scaffolding of the readily dissolvable cartridge complex.

In some embodiments, process **300** includes a step of deploying at least one stencil, in which an exterior outline of at least one stencil essentially conforms and/or matches with a shape of axial partitions of the segmental scaffolding, extending transversally to and along the longitudinal centerline of the dissolvable cartridge complex. In some embodiments, process **300** includes step of deploying at least one stencil, in which an exterior outline of at least one stencil essentially conforms and/or matches with a shape of the structured payload substance pellets.

In some embodiments, process **300** includes a step of saturating the payload substance and the effervescent material, with a predefined amount of saturating substrate, thereby conferring the payload substance and the effervescent material a semi-liquefied consistency. The term semi-liquefied consistency comprises a slurry and/or sludge like consistency. In some embodiments, process **300** includes a step of controllably delivering the payload substance and the effervescent material, into a predetermined location within the form.

In some embodiments, process **300** comprises step **314** of iteratively forming a plurality of segments of the readily dissolvable cartridge complex separated by the radial partitions.

In some embodiments, process **300** further includes a step of furnishing the readily dissolvable cartridge complex with a coating configured to absorb and/or neutralize undesired additives or impurities in water. In some embodiments, process **300** includes further a step of furnishing the readily dissolvable cartridge complex with a coating comprising water softening agent, including natrium chloride.

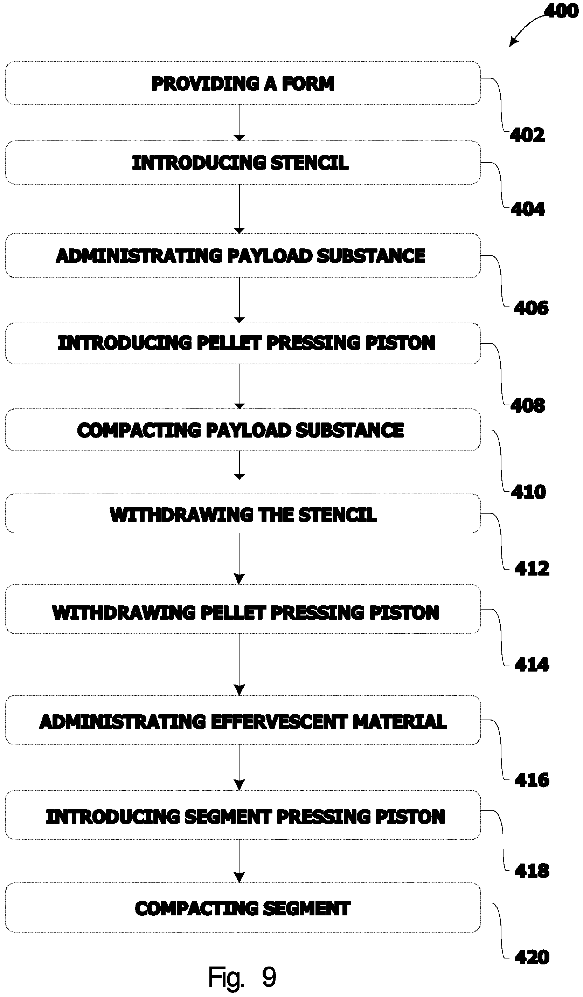

In accordance with some embodiments of the present invention, reference is now made **FIG 9** showing a flowchart of industrial process **400** of essentially dry fabricating of the readily dissolvable cartridge. The process of the embodiment of **FIG 9** illustrates various features that may be interchangeable with elements of any other embodiment described in the specification.

In some embodiments, process **400** commences with step **402** of providing a form, such as form **102** shown in **FIG 6A** and **6B****.** In some embodiments, process **400** further comprises step **404** of introducing a stencil, such as stencil **124** shown in **FIG 6A** and **6B****,** into the form.

In some embodiments, process **400** further includes step **406** of administrating the payload substance into the form. Step **406** typically comprises flattening and/or leveling the payload substance using vibration and/or a dedicated tool and/or conveying mini screws and/or air pressure and/or mechanical pressure. In some embodiments, process **400** further comprises step **408** of introducing a pellet pressing piston, such as pellet pressing piston array **128** shown in **FIG 6A** and **6B****,** into the form. The pellet pressing piston essentially the exterior outline of the structured payload substance pellet.

In some embodiments, process **400** further comprises step **410** of compacting the payload substance, thereby forming the plurality of structured payload substance pellets, within one segment. In some embodiments, step **406** of administrating the payload substance into the form as well as step **410** of compacting the payload substance are performed in combination and *a priori,* so that the plurality of structured payload substance pellets are pre-pressed ahead, so that already compacted structured payload substance pellets are administered into the form.

In some embodiments, process **400** further includes step **412** of withdrawing the stencil from the form. In some embodiments, process **400** further comprises step **414** of withdrawing the pellet pressing piston from the form. In some embodiments, process **400** further includes step **416** of administrating an effervescent material into the form, thereby filling the void spaces between the structured payload substance pellets thereby forming the axial partitions between the pellets, as well as additionally filling a layer above the structured payload substance pellets in the constructed segment, thereby providing material for the radial partition, such as radial partitions **16** shown in **FIG 3A****,** between the segments. In some embodiments, step **416** further comprises step of flattening and/or leveling the effervescent material using vibration and/or a dedicated tool and/or conveying mini screws and/or air pressure and/or mechanical pressure.

In some embodiments, process **400** further comprises step **418** of introducing a segment pressing piston, such as segment pressing piston **130** shown in **FIG 6A****,** into the form. The segment pressing piston assumes essentially the entire cross-sectional area of the form. In some embodiments process **400** includes step **420** of compacting, thereby forming a segment within the readily dissolvable cartridge complex. In some embodiments, process **400** yet further comprises a step of withdrawing the segment pressing piston from the form. In some embodiments, process **400** ultimately includes an iterative step of repeating steps **404 to 420.**

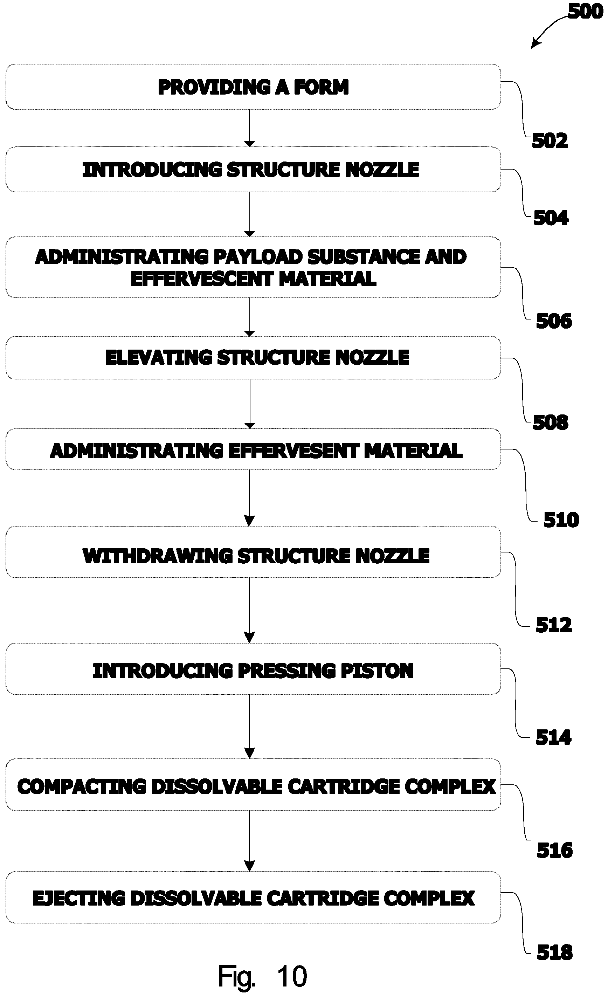

In accordance with another embodiment of the present invention, reference is now made **FIG 10** showing a flowchart of industrial process **500** of essentially dry fabricating of the readily dissolvable cartridge. The process of the embodiment of **FIG 10** illustrates various features that may be interchangeable with elements of any other embodiment described in the specification.

In some embodiments, process **500** commences with step **502** of providing a form, such as form **202** shown in **FIG 7A to 7I****.** In some embodiments, process **500** further comprises step **504** of introducing a structured nozzle, such as structured nozzle **204** shown in **FIG 7A** to **7I**, into the form. The structured nozzle comprises a plurality of dedicated payload substance nozzles and an effervescent material nozzle.

In some embodiments, process **500** further includes step **506** of administrating, optionally simultaneously, the payload substance and the effervescent material into the form, via the structured nozzle. Step **506** typically comprises flattening and/or leveling the payload substance using vibration and/or a dedicated tool and/or conveying mini screws and/or air pressure and/or mechanical pressure. In some embodiments, process **500** further comprises step **508** of elevating the structured nozzle by axial translation of the structured nozzle within the form in an upward direction, thereby forming a segment of the readily dissolvable cartridge complex.

In some embodiments, process **500** further comprises step **510** of administrating a layer of effervescent material, optionally from the effervescent material nozzle, while elevating by axial translation of the structured nozzle within the form in an upward direction and optionally rotating the structured nozzle, thereby providing material for the radial partition, such as radial partition **16** shown in **FIG 3A****,** between the segments. Step **510** typically comprises flattening and/or leveling the effervescent material. In some embodiments, process **500** ultimately includes an iterative step of repeating steps **506 to 510.**

In some embodiments, process **500** further comprises step **512** of withdrawing the structured nozzle from the form once the entire readily dissolvable cartridge complex and/or a single segment of the readily dissolvable cartridge complex is formed. In some embodiments, process **500** further comprises step **514** of introducing a pressing piston, such as segment pressing piston **130** shown in **FIG 6A** and **6B****,** into the form. The segment pressing piston typically assumes essentially the entire cross-sectional area of the form.

In some embodiments, process **500** further comprises step **516** of compacting the readily dissolvable cartridge complex. In some embodiments, process **500** further concluded with step **518** of ejecting the readily dissolvable cartridge complex from the form.

## Claims

1. An industrial process of fabricating a readily dissolvable cartridge complex comprises:
(a) preparing an effervescent material readily dissolvable in an aqueous solution;
(b) forming at least one segment of a segmental scaffolding from said effervescent material comprising:
(I) forming at least on radial partition of said at least one segment of said segmental scaffolding, extending about said longitudinal centerline of said dissolvable cartridge complex;
(II) forming a plurality of axial partitions within said at least one segment of said segmental scaffolding, extending transversally to and along said longitudinal centerline of said dissolvable cartridge complex;
(III) forming a plurality of compartments formed in-between said axial partitions, in each one of said plurality of said segments of said segmental scaffolding;
(c) preparing a payload substance;
(d) forming a plurality of structured payload substance pellets;
(e) accommodating said structured payload substance pellets in said compartments of said segmental scaffolding;
(f) compressing at least one segment of said segmental scaffolding of said readily dissolvable cartridge complex;
(g) iteratively forming a plurality of segments of said readily dissolvable cartridge complex separated by said radial partitions.

2. The industrial process, as in claim 1, further comprises furnishing said readily dissolvable cartridge complex with a coating configured to absorb and/or neutralize undesired additives or impurities in water.

3. The industrial process, as in claim 1, further comprises a furnishing said readily dissolvable cartridge complex with a coating comprising water softening agent, including natrium chloride.

4. The industrial process, as in claim 1, further comprises controllably saturating at least one member selected from the group consisting of: said payload substance and said effervescent material, with a predefined amount of saturating substrate, thereby conferring to said at least one member a semi-liquefied consistency.

5. The industrial process, as in claim 1, further comprises:
(a) controllably saturating said payload substance and said effervescent material, thereby conferring thereto a semi-liquefied consistency, and
(b) controllably delivering said payload substance and said effervescent material, into a predetermined location within said form.

6. The industrial process, as in claim 1, further comprises deploying at least one stencil, wherein an exterior outline of said at least one stencil essentially matches at least one shape selected from the group consisting of:
(a) a shape of axial partitions of said segmental scaffolding, extending transversally to and along said longitudinal centerline of said dissolvable cartridge complex;
(b) a shape of said structured payload substance pellets.

7. The industrial process as in claim 1, wherein said compressing comprises essentially matches with exterior outline of a shape of said structured payload substance pellets.

8. An industrial manufacture system for fabricating a readily dissolvable cartridge complex comprises:
(a) at least one form, comprising an essentially hollow shape, wherein an interior surface of said at least one form essentially matches with a shape of said readily dissolvable cartridge complex;
(b) a segmental scaffolding fabricating machine comprising:
(I) an effervescent material reservoir, containing an effervescent material readily dissolvable in an aqueous solution;
(II) a dossing module connected to said reservoir of effervescent material, configured to receive said effervescent material from said reservoir of effervescent material and to portion a predetermined amount of said effervescent material;
(III) a fabricating module comprising at least one nozzle, configured to dispose said effervescent material in a predefined structured arrangement within said form, thereby forming a plurality of compartments, within at least one segment of said segmental scaffolding;
(IV) a compacting mechanism, comprising at least one pressing piston operationally connected to a pressure exerting device, configured to compress at least one segment of said segmental scaffolding of said readily dissolvable cartridge complex within said form;
(c) a structured payload substance pellets fabricating machine comprising:
(I) a payload substance reservoir, containing a payload substance;
(II) a dossing module connected to said reservoir of payload substance, configured to receive said payload substance from said reservoir of payload substance and to portion a predetermined amount of said payload substance;
(III) a fabricating module comprising at least one nozzle, configured to dispose said payload substance in a predefined structured arrangement within said form, thereby forming a plurality of structured payload substance pellets within said compartments in at least one segment of said segmental scaffolding;
(IV) a compacting mechanism, comprising at least one pressing piston operationally connected to a pressure exerting device, configured to compress said structured payload substance pellets within said compartments, thereby accommodating said structured payload substance pellets in said compartments, formed within at least one segment of said segmental scaffolding of said readily dissolvable cartridge complex, within said form.

9. The industrial manufacture system, as in claim 8, further comprises a saturating module, configured for controllably saturate at least one member selected from the group consisting of: said payload substance and said effervescent material, with a predefined amount of saturating substrate, thereby conferring to said at least one member a semi-liquefied consistency.

10. The industrial manufacture system, as in claim 8, further comprises a pulverizator, configured for furnishing said readily dissolvable cartridge complex with a coating configured to absorb and/or neutralize undesired additives or impurities in water.

11. The industrial manufacture system as, in claim 8, further comprises at least one stencil, wherein an exterior outline of said at least one stencil essentially matches with at least one shape selected from the group consisting of:
(a) a shape of axial partitions of said segmental scaffolding, extending transversally to and along said longitudinal centerline of said dissolvable cartridge complex;
(b) a shape of said structured payload substance pellets.

12. The industrial manufacture system as in claim 8, wherein said compacting mechanism of said structured payload substance pellets fabricating machine comprises a plurality of pressing pistons, wherein an exterior outline of said pistons essentially matches with exterior outline of a shape of said structured payload substance pellets.

13. A readily dissolvable cartridge complex comprises:
(a) a segmental scaffolding comprising an effervescent material readily dissolvable in an aqueous solution;
(b) a plurality of radial partitions of said segmental scaffolding, arranged in tandem, extending about said longitudinal centerline of said dissolvable cartridge complex;
(c) a plurality of segments of said dissolvable cartridge complex, separated by said radial partitions and formed in-between said radial partitions;
(d) a plurality of axial partitions of said segmental scaffolding, extending transversally to and along said longitudinal centerline of said dissolvable cartridge complex, dividing each one of said plurality of said segments of said dissolvable cartridge complex into a plurality of sectors;
(e) a plurality of compartments formed in-between said axial partitions within said sectors, in each one of said plurality of said segments of said dissolvable cartridge complex;
(f) a plurality of structured payload pellets, accommodated in said compartments.

14. The readily dissolvable cartridge complex, as in claim 13, further comprises a coating configured to absorb and/or neutralize undesired additives or impurities in water.

15. The readily dissolvable cartridge complex, as in claim 13, further comprises a coating comprising a water softening agent.

## Patentansprüche

1. Industrieller Prozess zum Herstellen eines leicht löslichen Kartuschenkomplexes, umfassend:
a) Zubereiten eines schäumenden Materials, das in einer wässerigen Lösung leicht löslich ist;
b) Bilden mindestens eines Segments eines segmentierten Gerüsts aus dem schäumenden Material, umfassend:
I) Bilden mindestens einer radialen Trennwand des mindestens einen Segments des segmentierten Gerüsts, die sich um die Längsmittellinie des löslichen Kartuschenkomplexes herum erstreckt;
II) Bilden einer Vielzahl von axialen Trennwänden in dem mindestens einen Segment des segmentierten Gerüsts, die sich quer bis zu und entlang der Längsmittellinie des löslichen Kartuschenkomplexes erstrecken;
III) Bilden einer Vielzahl von Fächern, die zwischen den axialen Trennwänden gebildet sind, in jedem der Vielzahl der Segmente des segmentierten Gerüsts;
c) Zubereiten einer Nutzsubstanz;
d) Bilden einer Vielzahl von strukturierten Nutzsubstanz-Presslingen;
e) Unterbringen der strukturierten Nutzsubstanz-Presslinge in den Fächern des segmentierten Gerüsts;
f) Komprimieren mindestens eines Segments des segmentierten Gerüsts des leicht löslichen Kartuschenkomplexes;
g) iteratives Bilden einer Vielzahl von Segmenten des leicht löslichen Kartuschenkomplexes, die durch die radialen Trennwände getrennt sind.

2. Industrieller Prozess nach Anspruch 1, ferner umfassend das Ausstatten des leicht löslichen Kartuschenkomplexes mit einer Beschichtung, die dazu konfiguriert ist, unerwünschte Zusatzstoffe und Verunreinigungen in Wasser zu absorbieren und/oder zu neutralisieren.

3. Industrieller Prozess nach Anspruch 1, ferner umfassend das Ausstatten des leicht löslichen Kartuschenkomplexes mit einer Beschichtung, die ein Wasserenthärtungsmittel umfasst, das Natriumchlorid umfasst.

4. Industrieller Prozess nach Anspruch 1, ferner umfassend das kontrollierbare Sättigen mindestens eines Elements, das aus der Gruppe ausgewählt wird, die besteht aus: der Nutzsubstanz und dem schäumenden Material, mit einer vordefinierten Menge des Sättigungssubstrats, wodurch dem mindestens einen Element eine halbverflüssigte Konsistenz verliehen wird.

5. Industrieller Prozess nach Anspruch 1, ferner umfassend:
a) kontrollierbares Sättigen der Nutzsubstanz und des schäumenden Materials, wodurch ihnen eine halbverflüssigte Konsistenz verliehen wird, und
b) kontrollierbares Abgeben der Nutzsubstanz und des schäumenden Materials an einer vorbestimmten Stelle innerhalb des Formwerkzeugs.

6. Industrieller Prozess nach Anspruch 1, ferner umfassend das Einsetzen mindestens eines Stempels, wobei der äußerer Umriss des mindestens einen Stempels im Wesentlichen mit mindestens einer Form übereinstimmt, die aus der Gruppe ausgewählt wird, die besteht aus:
a) einer Form der axialen Trennwände des segmentierten Gerüsts, die sich quer bis zu und entlang der Längsmittellinie des löslichen Kartuschenkomplexes erstrecken;
b) einer Form der strukturierten Nutzsubstanz-Presslinge.

7. Industrieller Prozess nach Anspruch 1, wobei das Komprimieren das wesentliche Übereinstimmen mit dem äußeren Umriss einer Form der strukturierten Nutzsubstanz-Presslinge umfasst.

8. Industrielles Herstellungssystem zum Herstellen eines leicht löslichen Kartuschenkomplexes, umfassend:
a) mindestens ein Formwerkzeug, das eine im Wesentlichen hohle Form umfasst, wobei eine innere Oberfläche des mindestens einen Formwerkzeugs im Wesentlichen mit einer Form des leicht löslichen Kartuschenkomplexes übereinstimmt;
b) eine Maschine zum Herstellen eines segmentierten Gerüsts, umfassend:
I) einen Behälter für schäumendes Material, der ein schäumendes Material, das in einer wässerigen Lösung leicht löslich ist, enthält;
II) ein Dosiermodul, das mit dem Behälter für schäumendes Material verbunden ist und dazu konfiguriert ist, das schäumende Material aus dem Behälter für schäumendes Material aufzunehmen und eine vorbestimmte Menge des schäumenden Materials zu portionieren;
III) ein Herstellungsmodul, das mindestens eine Düse umfasst, die dazu konfiguriert ist, das schäumende Material in einer vordefinierten strukturierten Anordnung in dem Formwerkzeug anzuordnen, wodurch eine Vielzahl von Fächern in mindestens einem Segment des segmentierten Gerüsts gebildet wird;
IV) einen Verdichtungsmechanismus, der mindestens einen Druckkolben umfasst, der betriebsfähig mit einer Druck ausübenden Vorrichtung verbunden ist, die dazu konfiguriert ist, mindestens ein Segment des segmentierten Gerüsts des leicht löslichen Kartuschenkomplexes in dem Formwerkzeug zu komprimieren;
c) eine Maschine zum Herstellen von strukturierten Nutzsubstanz-Presslingen, umfassend:
I) einen Nutzsubstanzbehälter, der eine Nutzsubstanz enthält;
II) ein Dosiermodul, das mit dem Nutzsubstanzbehälter verbunden ist und dazu konfiguriert ist, die Nutzsubstanz aus dem Nutzsubstanzbehälter aufzunehmen und eine vorbestimmte Menge der Nutzsubstanz zu portionieren;
III) ein Herstellungsmodul, das mindestens eine Düse umfasst, die dazu konfiguriert ist, die Nutzsubstanz in einer vordefinierten strukturierten Anordnung in dem Formwerkzeug anzuordnen, wodurch eine Vielzahl von strukturierten Nutzsubstanz-Presslingen in den Fächern in mindestens einem Segment des segmentierten Gerüsts gebildet wird;
IV) einen Verdichtungsmechanismus, der mindestens einen Druckkolben umfasst, der betriebsfähig mit einer Druck ausübenden Vorrichtung verbunden ist, die dazu konfiguriert ist, die strukturierten Nutzsubstanz-Presslinge in den Fächern zu komprimieren, wodurch die strukturierten Nutzsubstanz-Presslinge in den Fächern untergebracht werden, die in mindestens einem Segment des segmentierten Gerüsts des leicht löslichen Kartuschenkomplexes in dem Formwerkzeug gebildet sind.

9. Industrielles Herstellungssystem nach Anspruch 8, ferner umfassend ein Sättigungsmodul, das dazu konfiguriert ist, mindestens ein Element kontrolliert zu sättigen, das aus der Gruppe ausgewählt wird, die besteht aus: der Nutzsubstanz und dem schäumenden Material, mit einer vordefinierten Menge des Sättigungssubstrats, wodurch dem mindestens einen Element eine halbverflüssigte Konsistenz verliehen wird.

10. Industrielles Herstellungssystem nach Anspruch 8, ferner umfassend ein Feinmahlwerk, das dazu konfiguriert ist, den leicht löslichen Kartuschenkomplex mit einer Beschichtung auszustatten, die dazu konfiguriert ist, unerwünschte Zusatzstoffe und Verunreinigungen in Wasser zu absorbieren und/oder zu neutralisieren.

11. Industrielles Herstellungssystem nach Anspruch 8, ferner umfassend mindestens einen Stempel, wobei ein äußerer Umriss des mindestens einen Stempels im Wesentlichen mit mindestens einer Form übereinstimmt, die aus der Gruppe ausgewählt wird, die besteht aus:
a) einer Form von axialen Trennwänden des segmentierten Gerüsts, die sich quer bis zu und entlang der Längsmittellinie des löslichen Kartuschenkomplexes erstrecken;
b) einer Form der strukturierten Nutzsubstanz-Presslinge.

12. Industrielles Herstellungssystem nach Anspruch 8, wobei der Verdichtungsmechanismus der Maschine zum Herstellen von strukturierten Nutzsubstanz-Presslingen eine Vielzahl von Druckkolben umfasst, wobei ein äußerer Umriss der Kolben im Wesentlichen mit dem äußeren Umriss einer Form der strukturierten Nutzsubstanz-Presslinge übereinstimmt.

13. Leicht löslicher Kartuschenkomplex, umfassend:
a) ein segmentiertes Gerüst, das ein schäumendes Material, das in einer wässerigen Lösung leicht löslich ist, umfasst;
b) eine Vielzahl von radialen Trennwänden des segmentierten Gerüsts, die hintereinander angeordnet sind und sich um die Längsmittellinie des löslichen Kartuschenkomplexes erstrecken;
c) eine Vielzahl von Segmenten des löslichen Kartuschenkomplexes, die durch die radialen Trennwände getrennt sind und zwischen den radialen Trennwänden gebildet sind;
d) eine Vielzahl von axialen Trennwänden des segmentierten Gerüsts, die sich quer zu und entlang der Längsmittellinie des löslichen Kartuschenkomplexes erstrecken, wobei sie jedes der Vielzahl der Segmente des löslichen Kartuschenkomplexes in eine Vielzahl von Sektoren unterteilen;
e) eine Vielzahl von Fächern, die zwischen den axialen Trennwänden in den Sektoren in jedem der Vielzahl der Segmente des löslichen Kartuschenkomplexes gebildet sind;
f) eine Vielzahl von strukturierten Nutzsubstanz-Presslingen, die in den Fächern untergebracht sind.

14. Leicht löslicher Kartuschenkomplex nach Anspruch 13, ferner umfassend eine Beschichtung, die dazu konfiguriert ist, unerwünschte Zusatzstoffe und Verunreinigungen in Wasser zu absorbieren und/oder zu neutralisieren.

15. Leicht löslicher Kartuschenkomplex nach Anspruch 13, ferner umfassend eine Beschichtung, die ein Wasserenthärtungsmittel umfasst.

## Revendications

1. Processus industriel permettant de fabriquer un complexe de cartouche facilement soluble, comprenant les étapes consistant à :
a) préparer une matière effervescente facilement soluble dans une solution aqueuse ;
b) former au moins un segment d'une ossature segmentée à partir de ladite matière effervescente, comprenant les étapes consistant à :
I) former au moins une partition radiale dudit au moins un segment de ladite ossature segmentée, s'étendant autour de ladite ligne médiane longitudinale dudit complexe de cartouche soluble ;
II) former une pluralité de partitions axiales à l'intérieur dudit au moins un segment de ladite ossature segmentée, s'étendant transversalement jusqu'à et le long de ladite ligne médiane longitudinale dudit complexe de cartouche soluble ;
III) former une pluralité de compartiments formés entre lesdites partitions axiales, dans chacun de ladite pluralité desdits segments de ladite ossature segmentée ;
c) préparer une substance de charge utile ;
d) former une pluralité de pastilles de substance de charge utile structurées ;
e) loger lesdites pastilles de substance de charge utile structurées dans lesdits compartiments de ladite ossature segmentée ;
f) comprimer au moins un segment de ladite ossature segmentée dudit complexe de cartouche facilement soluble ;
g) former de manière itérative une pluralité de segments dudit complexe de cartouche facilement soluble, séparés par lesdites partitions radiales.

2. Processus industriel selon la revendication 1, comprenant en outre l'équipement dudit complexe de cartouche facilement soluble d'un enrobage configuré pour absorber et/ou neutraliser des additifs ou impuretés indésirables dans l'eau.

3. Processus industriel selon la revendication 1, comprenant en outre l'équipement dudit complexe de cartouche facilement soluble d'un enrobage comprenant agent adoucisseur d'eau, comprenant du chlorure de sodium.

4. Processus industriel selon la revendication 1, comprenant en outre la saturation contrôlable d'au moins un élément sélectionné dans le groupe composé : de ladite substance de charge utile et de ladite matière effervescente, avec une quantité prédéfinie d'un substrat de saturation, conférant ainsi une consistance semi-liquéfiée audit au moins un élément.

5. Processus industriel selon la revendication 1, comprenant en outre :
a) la saturation contrôlable de ladite substance de charge utile et de ladite matière effervescente, leur conférant ainsi une consistance semi-liquéfiée, et
b) la distribution contrôlable de ladite substance de charge utile et de ladite matière effervescente dans un endroit prédéterminé à l'intérieur dudit moule.

6. Processus industriel selon la revendication 1, comprenant en outre le déploiement d'au moins un poinçon, dans lequel un contour extérieur dudit au moins un poinçon correspond substantiellement à au moins une forme sélectionnée dans le groupe composé :
a) d'une forme de partitions axiales de ladite ossature segmentée, s'étendant transversalement à et le long de ladite ligne médiane longitudinale dudit complexe de cartouche soluble ;
b) d'une forme desdites pastilles de substance de charge utile structurées.

7. Processus industriel selon la revendication 1, dans lequel ladite compression comprend la correspondance substantielle au contour extérieur d'une forme desdites pastilles de substance de charge utile structurées.

8. Système de fabrication industriel permettant de fabriquer un complexe de cartouche facilement soluble, comprenant :
a) au moins un moule, ayant une forme substantiellement creuse, dans lequel une surface intérieure dudit au moins un moule correspond substantiellement à une forme dudit complexe de cartouche facilement soluble ;
b) une machine de fabrication d'une ossature segmentée, comprenant :
I) un réservoir de matière effervescente, contenant une matière effervescente facilement soluble dans une solution aqueuse ;
II) un module de dosage connecté audit réservoir de matière effervescente, configuré pour recevoir ladite matière effervescente dudit réservoir de matière effervescente et pour mettre en portions une quantité prédéterminée de ladite matière effervescente ;
III) un module de fabrication comprenant au moins une buse, configurée pour disposer ladite matière effervescente dans un agencement structuré prédéfini à l'intérieur dudit moule, formant ainsi une pluralité de compartiments, à l'intérieur d'au moins un segment de ladite ossature segmentée ;
IV) un mécanisme de compactage, comprenant au moins un piston de pression connecté de manière opérationnelle à un dispositif exerçant de la pression, configuré pour comprimer au moins un segment de ladite ossature segmentée dudit complexe de cartouche facilement soluble à l'intérieur dudit moule ;
c) une machine de fabrication de pastilles de substance de charge utile structurées, comprenant :
I) un réservoir de substance de charge utile, contenant une substance de charge utile ;
II) un module de dosage connecté audit réservoir de substance de charge utile, configuré pour recevoir ladite substance de charge utile dudit réservoir de substance de charge utile et pour mettre en portions une quantité prédéterminée de ladite substance de charge utile ;
III) un module de fabrication comprenant au moins une buse, configurée pour disposer ladite substance de charge utile dans un agencement structuré prédéfini à l'intérieur dudit moule, formant ainsi une pluralité de pastilles de substance de charge utile structurées à l'intérieur desdits compartiments dans au moins un segment de ladite ossature segmentée ;
IV) un mécanisme de compactage, comprenant au moins un piston de pression connecté de manière opérationnelle à un dispositif exerçant de la pression, configuré pour comprimer lesdites pastilles de substance de charge utile structurées à l'intérieur desdits compartiments, logeant ainsi lesdites pastilles de substance de charge utile structurées dans lesdits compartiments, formés à l'intérieur d'au moins un segment de ladite ossature segmentée dudit complexe de cartouche facilement soluble, à l'intérieur dudit moule.

9. Système de fabrication industriel selon la revendication 8, comprenant en outre un module de saturation, configuré pour saturer de manière contrôlée au moins un élément sélectionné dans le groupe composé : de ladite substance de charge utile et de ladite matière effervescente, avec une quantité prédéfinie de substrat de saturation, conférant ainsi audit au moins un élément une consistance semi-liquéfiée.

10. Système de fabrication industriel selon la revendication 8, comprenant en outre un pulvérisateur, configuré pour équiper ledit complexe de cartouche facilement soluble d'un enrobage configuré pour absorber et/ou neutraliser des additifs ou impuretés indésirables dans l'eau.

11. Système de fabrication industriel selon la revendication 8, comprenant en outre au moins un poinçon, dans lequel un contour extérieur dudit au moins un poinçon correspond substantiellement à au moins une forme sélectionnée dans le groupe composé :
a) d'une forme de partitions axiales de ladite ossature segmentée, s'étendant transversalement à et le long de ladite ligne médiane longitudinale dudit complexe de cartouche soluble ;
b) d'une forme desdites pastilles de substance de charge utile structurées.

12. Système de fabrication industriel selon la revendication 8, dans lequel ledit mécanisme de compactage de ladite machine de fabrication de pastilles de substance de charge utile structurées comprend une pluralité de pistons de pression, dans lequel un contour extérieur desdits pistons correspond substantiellement au contour extérieur d'une forme desdites pastilles de substance de charge utile structurées.

13. Complexe de cartouche facilement soluble, comprenant :
a) une ossature segmentée comprenant une matière effervescente facilement soluble dans une solution aqueuse ;
b) une pluralité de partitions radiales de ladite ossature segmentée, agencées en tandem, s'étendant autour de ladite ligne médiane longitudinale dudit complexe de cartouche soluble ;
c) une pluralité de segments dudit complexe de cartouche soluble, séparés par lesdites partitions radiales et formés entre lesdites partitions radiales ;
d) une pluralité de partitions axiales de ladite ossature segmentée, s'étendant transversalement à et le long de ladite ligne médiane longitudinale dudit complexe de cartouche soluble, divisant chacun de ladite pluralité desdits segments dudit complexe de cartouche soluble en une pluralité de secteurs ;
e) une pluralité de compartiments formés entre lesdites partitions axiales à l'intérieur desdits secteurs, dans chacun de ladite pluralité desdits segments dudit complexe de cartouche soluble ;
f) une pluralité de pastilles de charge utile structurées, logées dans lesdits compartiments.

14. Complexe de cartouche facilement soluble selon la revendication 13, comprenant en outre un enrobage configuré pour absorber et/ou neutraliser des additifs ou impuretés indésirables dans l'eau.

15. - Complexe de cartouche facilement soluble selon la revendication 13, comprenant en outre un enrobage comprenant un agent adoucisseur d'eau.
